(19) Europäisches Patentamt

European Patent Office

Office européen des brevets

(11) **EP 1 259 521 B1**

(12) **FASCICULE DE BREVET EUROPEEN**

(45) Date de publication et mention
de la délivrance du brevet:
**29.12.2004 Bulletin 2004/53**

(51) Int Cl.⁷: **C07F 9/94**, C07C 309/06

(21) Numéro de dépôt: **01911835.5**

(86) Numéro de dépôt international:
**PCT/FR2001/000630**

(22) Date de dépôt: **02.03.2001**

(87) Numéro de publication internationale:
**WO 2001/064695 (07.09.2001 Gazette 2001/36)**

(54) **PROCEDE DE PREPARATION DU TRIS-TRIFLUOROMETHANESULFONATE DE BISMUTH**

VERFAHREN ZUR HERSTELLUNG VON WISMUTH TRIS-TRIFLUORMETHANSULFONAT

METHOD FOR PREPARING BISMUTH TRIS-TRIFLUOROMETHANESULPHONATE

(84) Etats contractants désignés:
**AT BE CH CY DE DK ES FI FR GB GR IE IT LI LU
MC NL PT SE TR**

(30) Priorité: **03.03.2000 FR 0002781**

(43) Date de publication de la demande:
**27.11.2002 Bulletin 2002/48**

(73) Titulaire: **CENTRE NATIONAL DE
LA RECHERCHE SCIENTIFIQUE (CNRS)
75016 Paris (FR)**

(72) Inventeurs:
 • **LE ROUX, Christophe
 F-36000 Chateauroux (FR)**
 • **REPICHET, Sigrid
 F-27400 LOUVIERS (FR)**
 • **DUBAC, Jacques, Roger, André
 F-31320 Pechbusque (FR)**

(74) Mandataire: **Bernasconi, Jean Raymond et al
c/o Cabinet Lavoix,
2, Place d'Estienne d'Orves
75441 Paris Cedex 09 (FR)**

(56) Documents cités:
**WO-A-97/11930      US-A- 5 773 637**

• **NIYOGI D. G.: "Some reactions of trifluoromethanesulfonic anhydride with various metal
substrates" JOURNAL OF FLUORINE
CHEMISTRY., vol. 48, no. 3, 1 juillet 1990
(1990-07-01), pages 421-428, XP002150988
ELSEVIER SEQUOIA. LAUSANNE., CH ISSN:
0022-1139**
• **FRANK W.: "The nonaaquabismuth (III) cation"
ANGEWANDTE CHEMIE. INTERNATIONAL
EDITION., vol. 34, no. 21, 17 novembre 1995
(1995-11-17), pages 2416-2417, XP002150989
VERLAG CHEMIE. WEINHEIM., DE ISSN:
0570-0833**
• **LABROUILLERE M ET AL: "An Efficient Method
for the Preparation of Bismuth(III)
Trifluoromethanesulfonate" TETRAHEDRON
LETTERS,NL,ELSEVIER SCIENCE
PUBLISHERS, AMSTERDAM, vol. 40, no. 2, 8
janvier 1999 (1999-01-08), pages 285-286,
XP004152532 ISSN: 0040-4039**
• **CHEMICAL ABSTRACTS, vol. 100, no. 2, 9
janvier 1984 (1984-01-09) Columbus, Ohio, US;
abstract no. 16729, SINGH, SUKHJINDER ET AL:
"Trifluoromethanesulfonates of antimony(III)
and bismuth(III)" XP002150991 & INDIAN J.
CHEM., SECT. A (1983), 22A(9), 814-15, 1983,**

**Description**

La présente invention a trait à un nouveau procédé de préparation du tris-trifluorométhanesulfonate de bismuth.

Le tris-trifluorométhanesulfonate de bismuth, souvent désigné sous la dénomination triviale de triflate de bismuth, est le composé de formule $Bi(OSO_2CF_3)_3$, noté également $Bi(OTf)_3$.

De façon générale, les triflates métalliques sont connus comme étant des acides de Lewis particulièrement intéressants et qui sont mis en oeuvre notamment à titre de catalyseurs dans de nombreuses réactions, en particulier dans le domaine de la chimie organique. A ce sujet, on pourra par exemple se reporter aux articles de Kobayashi dans *Synlett*, pages 689-701 (1994), de Marshman dans *Aldrichimica Acta,* volume 28, pages 77-84 (1995), de Noyori et al. dans *Tetrahedron,* volume 37, pages 3899-3910 (1981) ou encore de Olah et al. dans le *Journal of the American Chemical Society,* volume 110, pages 2560-2565 (1988).

Le triflate de bismuth $Bi(OTf)_3$ présente la particularité intéressante d'être stable dans l'eau. Cette caractéristique particulière permet sa mise en oeuvre dans des catalyses en milieu aqueux, ce qui présente entre autres le double avantage d'effectuer les réactions dans un milieu respectueux de l'environnement et de pouvoir recycler le catalyseur à l'issu de la réaction.

Par ailleurs, il est à souligner que le bismuth est le moins toxique des éléments lourds, à l'inverse notamment du plomb, du mercure ou du thallium. De plus, contrairement par exemple aux terres rares, le bismuth est peu onéreux. En termes de coût, de toxicité et de répercussions sur l'environnement, $Bi(OTf)_3$ constitue de ce fait l'un des meilleurs triflates métalliques utilisables en catalyse.

De ce fait, $Bi(OTf)_3$ peut être utilisé industriellement, notamment dans la catalyse de réactions de Friedel-Crafts, et en particulier pour produire des cétones aromatiques avec de forts tonnages, comme cela est décrit par exemple dans les demandes de brevets WO 98/40 339 ou WO 97/11930.

Toutefois, les procédés de synthèse actuels de $Bi(OTf)_3$ sont relativement lourds et onéreux. En effet, pour obtenir quantitativement $Bi(OTf)_3$, les procédés conventionnels mettent généralement en oeuvre la réaction d'un large excès d'acide trifluorométhanesulfonique et/ou d'anhydride trifluorométhanesulfonique sur un composé du bismuth, tel que notamment l'oxyde de bismuth ou ses dérivés, comme par exemple le tris-trifluoroacétate de bismuth.

De tels procédés sont notamment décrits dans les articles de FRANK ("The nonaquabismuth (III) cation") pour dans ANGEWANDTE CHEMIE - INT. EDITION, vol. 4, n°21, pp. 2416-2417 (1995) ou de NIYOGI et al., paru dans le Journal of fluorine Chemistry, vol 48, no 3, pp. 421 - 428 (1990).

Or, l'acide trifluorométhanesulfonique TfOH, dit également acide triflique, est un produit coûteux, de même que l'anhydride trifluorométhanesulfonique $Tf_2O$ (anhydride triflique) correspondant.

De plus, les réactions de synthèse de $Bi(OTf)_3$ mettant en oeuvre ces composés nécessitent souvent des temps de réaction relativement longs.

Par ailleurs, l'utilisation de dérivés du bismuth autres que l'oxyde de bismuth conduit à la production de sous-produits qui peuvent poser des problèmes en termes de purification du produit final et éventuellement de répercussions sur l'environnement.

On a maintenant découvert que la mise en oeuvre de la réaction de l'acide triflique TfOH avec le trioxyde de bismuth $Bi_2O_3$ au sein d'un mélange eau/alcool mène de façon surprenante à une réaction quantitative de l'acide avec l'oxyde, et ce dans des conditions douces et avec des temps de réaction relativement courts par rapport aux autres procédés connus.

Sur la base de cette découverte, un premier but de la présente invention est de fournir un procédé de préparation du triflate de bismuth possédant un rendement élevé, sans que la mise en oeuvre d'un fort excès de réactif triflique ne soit nécessaire.

Un second but de l'invention consiste à fournir une méthode de synthèse de $Bi(OTf)_3$ nécessitant un temps de réaction réduit.

La présente invention a également pour but de fournir un procédé de synthèse du triflate de bismuth intéressant à la fois en termes de coûts et de répercussions sur l'environnement.

La présente invention a en effet pour objet un procédé de préparation du tris-trifluorométhanesulfonate de bismuth, caractérisé en ce qu'on fait réagir de l'acide trifluorométhanesulfonique $CF_3SO_3H$ avec du trioxyde de bismuth $Bi_2O_3$ dans un milieu constitué d'un mélange eau/alcool, et en ce qu'on récupère le tris-trifluorométhanesulfonate de bismuth obtenu.

Plus précisément, le procédé de l'invention met en oeuvre la réaction dont l'équation-bilan est la suivante :

$$Bi_2O_3 + 6\ TfOH \rightarrow 2\ Bi(OTf)_3 + 3\ H_2O \qquad \text{(équation I)}$$

Cette réaction de l'acide triflique avec l'oxyde de bismuth mise en oeuvre selon l'invention est avantageusement conduite dans un milieu constitué par un mélange d'eau et d'un alcool.

A ce sujet, il est à noter que, du fait de la mise en oeuvre d'un milieu réactionnel hydro-alcoolique dans le procédé de l'invention, le triflate de bismuth obtenu à l'issu de l'étape de réaction de TfOH avec $Bi_2O_3$ est spécifiquement un triflate de bismuth sous une forme hydratée.

Avantageusement, l'alcool mis en oeuvre dans le mélange réactionnel est un alcool miscible à l'eau et possédant de 1 à 3 atomes de carbone.

Notamment du fait de son coût peu élevé et de sa faible toxicité, l'alcool utilisé de façon préférentielle dans le mélange eau/alcool mis en oeuvre dans le procédé de l'invention est l'éthanol. Par ailleurs, il est également à noter que l'utilisation de l'éthanol conduit à des résultats particulièrement intéressants en termes de rendement et de vitesse de réaction.

De façon générale, pour obtenir à la fois un rendement élevé et une vitesse de réaction suffisante pour la réaction de TfOH et de $Bi_2O_3$, l'alcool, quel qu'il soit, est généralement présent en quantité majoritaire au sein du mélange spécifique eau/alcool mis en oeuvre.

Plus précisément, le rapport volumique alcool/eau au sein du milieu réactionnel initial, établi sur la base des volumes mesurés avant le mélange, est avantageusement compris entre 50:50 et 90:10, de préférence entre 60:40 et 80:20. De façon particulièrement préférée, ce rapport volumique possède une valeur proche du rapport 75:25.

L'utilisation du mélange particulier eau/alcool à titre de milieu réactionnel permet de mettre en oeuvre l'acide triflique et le trioxyde de bismuth dans des proportions proches de la stoechiométrie correspondant à l'équation I.

De fait, les réactifs sont généralement mis en oeuvre dans le procédé de l'invention avec un rapport molaire initial $TfOH/Bi_2O_3$ proche de 6:1, c'est-à-dire avantageusement compris entre 6:1 et 6,5:1 , et de préférence entre 6: 1 et 6,1:1.

Par ailleurs, de façon à mener à des conditions optimales en terme de rendement et de vitesse de réaction, la teneur en $Bi_2O_3$ au sein de la suspension initiale est généralement comprise entre 40 et 80 g/l, et de préférence entre 50 et 70 g/l.

De même, la concentration initiale en acide triflique dans le milieu est avantageusement comprise entre 70 et 150 g/l, et de façon préférentielle entre 100 et 130 g/l.

Il est également à noter que, compte tenu de l'état solide du trioxyde de bismuth et de son immiscibilité dans le milieu réactionnel eau/alcool, la réaction est généralement conduite sous agitation, de façon à former une suspension initiale et à optimiser les contacts solide/liquide au cours de la réaction.

D'autre part, de façon à obtenir un rendement élevé avec un temps de réaction relativement court, la réaction de TfOH avec $Bi_2O_3$ est généralement conduite à une température comprise entre 40 et 75°C, et de préférence entre 60 et 70°C.

Dans les conditions de concentrations et de température précitées, les temps de réaction observés pour la réaction de l'acide triflique avec le trioxyde de bismuth sont généralement compris entre 2 heures et 6 heures. De ce fait, la réaction mise en oeuvre dans le procédé de l'invention est généralement conduite pendant une durée allant de 2 heures à 4 heures.

Le rendement de la réaction de conversion de $Bi_2O_3$ en $Bi(OTf)_3$ observé lors de la mise en oeuvre du mélange particulier eau/alcool en tant que milieu réactionnel est généralement supérieur ou égal à 97%.

De plus, outre ces avantages en termes de rendement et de vitesse de réaction, le procédé de l'invention présente également les avantages suivants :

- la réaction de synthèse de $Bi(OTf)_3$ utilisée ne mène pas à la formation de sous-produits, hormis l'eau. Si on tient compte en outre de la mise en oeuvre d'un mélange de type eau/alcool à titre de milieu réactionnel, il apparaît que le procédé de l'invention s'inscrit tout à fait dans une logique de respect de l'environnement ;

- les conditions de réactions (température peu élevée, solvants peu toxiques, voire non toxiques) conduisent également à un procédé intéressant du point de vue de la sécurité ;

- l'isolement du produit de la réaction peut, du fait de l'absence de sous-produit et de la mise en oeuvre du mélange particulier eau/alcool, s'effectuer à l'aide de techniques bien connues, simples et peu coûteuses à mettre en oeuvre, telles que par exemple l'évaporation des solvants et la lyophilisation.

De fait, la récupération du triflate de bismuth produit au cours de la réaction de TfOH et $Bi_2O_3$ est généralement conduite par élimination du solvant et purification du produit, notamment par évaporation et lyophilisation. Le tris-trifluorométhanesulfonate de bismuth ainsi récupéré est généralement obtenu sous une forme hydratée.

L'objet de l'invention apparaîtra encore plus nettement au vu de l'exemple illustratif exposé ci-dessous.

**Exemple :**

Dans un ballon, on a introduit 2,293 g (soit 4,921 mmoles) de trioxyde de bismuth $Bi_2O_3$. On a ensuite additionné 35 ml d'un mélange éthanol+eau caractérisé par un rapport volumique éthanol/eau de 75:25. On a ensuite ajouté 4,452 g (soit 29,666 mmoles) d'acide triflique.

On a alors obtenu une suspension jaune qui a été soumise à une agitation à une température de 65°C pendant une durée de 3 heures, jusqu'à obtention d'une solution blanche laiteuse.

Les solvants (eau et éthanol) ont ensuite été éliminés sous pression réduite. Le solide blanc obtenu à l'issu de cette étape d'élimination de solvant a alors été dissous dans 30 ml d'eau distillée et soumis à une opération de lyophilisation dans un lyophilisateur de type Christ Alpha 2-4 (pression de 0,035 mbar (3500 Pa)), avec une température de réfrigération de -83°C.

De la sorte, on a récupéré 6,727 g d'un solide blanc.

Le solide obtenu présente les mêmes caractéristiques spectroscopiques (Résonance Magnétique Nucléaire du carbone et du fluor ; spectroscopie Infra-Rouge) qu'un échantillon de référence de $Bi(OTf)_3$ préparé selon la méthode décrite dans *Tetrahedron Letters*, volume 40, pages 285-286 (1999), c'est à dire les caractéristiques suivantes :

- RMN $^{13}C$ (50,3MHz,acétone-$d_6$) : δ =120 ppm (référence TMS)
  (quad., $^1J_{13C/19F}$ = 321 Hz) ;

- RMN $^{19}F$ (75.4MHz,acétone-$d_6$) : δ = 0,84 ppm (référence $CF_3COOH$);

- Infra Rouge (nujol) :ν = 3450-3550 $cm^{-1}$(m), 1230-1290(vs), 1180(s),
  1034(s), 1028(sh), 650(sh), 643(s).

L'analyse par diffractométrie de rayons X de la poudre blanche obtenue (enregistré sur un diffractomètre à haute résolution de type Siemens D500 en utilisant la raie $K\alpha_1$ du cuivre), à la température de 45°C, est par ailleurs en accord avec la structure du triflate de bismuth tétrahydraté [$Bi(OTf)_3$ ; $4H_2O$] publiée notamment dans *Chemical Materials*, volume 9, pages 3012-3016 (1997). La masse obtenue (6,727 g) correspond à un taux d'hydratation massique de 7%. Ce taux d'hydratation du triflate de bismuth obtenu est estimé par Résonance Magnétique Nucléaire du proton (solvant THF-$d_8$) en mesurant la quantité d'eau contenue dans une masse connue de triflate de bismuth (δ=11,3 ppm), en prenant comme référence le signal d'une quantité connue d'iodoforme (δ=5,2 ppm). Ainsi, le rendement de cette réaction, défini par le rapport du nombre de moles de triflate de bismuth obtenu sur deux fois le nombre de moles de trioxyde de bismuth introduit, est égal à 97 %.

**Revendications**

1. Procédé de préparation du tris-trifluorométhanesulfonate de bismuth, **caractérisé en ce qu'**on fait réagir de l'acide trifluorométhanesulfonique $CF_3SO_3H$ avec du trioxyde de bismuth $Bi_2O_3$ dans un milieu constitué d'un mélange eau/alcool, et **en ce qu'**on récupère le tris-trifluorométhanesulfonate de bismuth obtenu.

2. Procédé de préparation du tris-trifluorométhanesulfonate de bismuth selon la revendication 1, **caractérisé en ce que** l'alcool mis en oeuvre dans le milieu réactionnel est un alcool miscible à l'eau et possédant de 1 à 3 atomes de carbone.

3. Procédé de préparation du tris-trifluorométhanesulfonate de bismuth selon la revendication 1 ou la revendication 2, **caractérisé en ce que** l'alcool mis en oeuvre dans le milieu réactionnel est l'éthanol.

4. Procédé de préparation du tris-trifluorométhanesulfonate de bismuth selon l'une quelconque des revendications 1 à 3, **caractérisé en ce que** le rapport volumique alcool/eau au sein du milieu réactionnel est initialement compris entre 50:50 et 90:10.

5. Procédé de préparation du tris-trifluorométhanesulfonate de bismuth selon l'une quelconque des revendications 1 à 4, **caractérisé en ce que** le rapport volumique alcool/eau au sein du milieu réactionnel est initialement compris entre 60:40 et 80:20.

6. Procédé de préparation du tris-trifluorométhanesulfonate de bismuth selon l'une quelconque des revendications 1 à 5, **caractérisé en ce que** l'acide trifluorométhanesulfonique et le trioxyde de bismuth sont mis en oeuvre avec

un rapport molaire $CF_3SO_3H/Bi_2O_3$ initial compris entre 6:1 et 6,5:1.

7. Procédé de préparation du tris-trifluorométhanesulfonate de bismuth selon l'une quelconque des revendications 1 à 6, **caractérisé en ce que** la teneur initiale en $Bi_2O_3$ au sein du milieu réactionnel est comprise entre 40 et 80 g/l.

8. Procédé de préparation du tris-trifluorométhanesulfonate de bismuth selon l'une quelconque des revendications 1 à 7, caractérisé en ce la concentration initiale en acide triflique au sein du milieu réactionnel est comprise entre 70 et 150 g/l.

9. Procédé de préparation du tris-trifluorométhanesulfonate de bismuth selon l'une quelconque des revendications 1 à 8, **caractérisé en ce que** la réaction de l'acide triflique avec le trioxyde de bismuth est conduite à une température comprise entre 40 et 75°C.

10. Procédé de préparation du tris-trifluorométhanesulfonate de bismuth selon l'une quelconque des revendications 1 à 9, **caractérisé en ce que** ledit tris-trifluorométhanesulfonate de bismuth récupéré est obtenu sous une forme hydratée.


**Patentansprüche**

1. Verfahren zur Herstellung von Wismuth-tris-trifluormethansulfonat, **dadurch gekennzeichnet, dass** man Trifluormethansulfonsäure $CF_3SO_3H$ mit Wismuthtrioxid $Bi_2O_3$ in einem aus einem Gemisch Wasser/Alkohol bestehenden Medium umsetzt und dass man das erhaltene Wismuth-tris-trifluormethansulfonat gewinnt.

2. Verfahren zur Herstellung von Wismuth-tris-trifluormethansulfonat nach Anspruch 1, **dadurch gekennzeichnet, dass** der in dem Reaktionsmedium eingesetzte Alkohol ein mit Wasser mischbarer Alkohol mit 1 bis 3 Kohlenstoffatomen ist.

3. Verfahren zur Herstellung von Wismuth-tris-trifluormethansulfonat nach Anspruch 1 oder Anspruch 2, **dadurch gekennzeichnet, dass** der in dem Reaktionsmedium eingesetzte Alkohol Ethanol ist.

4. Verfahren zur Herstellung von Wismuth-tris-trifluormethonsulfonat nach einem der Ansprüche 1 bis 3, **dadurch gekennzeichnet, dass** das Volumenverhältnis Alkohol/Wasser in dem Reaktionsmedium anfangs zwischen 50:50 und 90:10 liegt.

5. Verfahren zur Herstellung von Wismuth-tris-trifluormethansulfonat nach einem der Ansprüche 1 bis 4, **dadurch gekennzeichnet, dass** das Volumenverhältnis Alkohol/Wasser in dem Reaktionsmedium anfangs zwischen 60:40 und 80:20 liegt.

6. Verfahren zur Herstellung von Wismuth-tris-trifluormethansulfonat nach einem der Ansprüche 1 bis 5, **dadurch gekennzeichnet, dass** man die Trifluormethansulfonsäure und das Wismuthtrioxid zu Beginn in einem Molverhältnis $CF_3SO_3H/Bi_2O_3$ von 6:1 bis 6,5:1, einsetzt.

7. Verfahren zur Herstellung von Wismuth-tris-trifluormethansulfonat nach einem der Ansprüche 1 bis 6, **dadurch gekennzeichnet, dass** der Anfangsgehalt an $Bi_2O_3$ in dem Reaktionsmedium zwischen 40 und 80 g/l liegt.

8. Verfahren zur Herstellung von Wismuth-tris-trifluormethansulfonat nach einem der Ansprüche 1 bis 7, **dadurch gekennzeichnet, dass** die Anfangskonzentration der Trifluormethansulfonsäure in dem Reaktionsmedium zwischen 70 und 150 g/l liegt.

9. Verfahren zur Herstellung von Wismuth-tris-trifluormethansulfonat nach einem der Ansprüche 1 bis 8, **dadurch gekennzeichnet, dass** die Reaktion der Trifluormethansulfonsäure mit dem Wismuthtrioxid bei einer Temperatur zwischen 40 und 75 °C durchgeführt wird.

10. Verfahren zur Herstellung von Wismuth-tris-trifluormethansulfonat nach einem der Ansprüche 1 bis 9, **dadurch gekennzeichnet, dass** das gewonne Wismuth-tris-trifluormethansulfonat in hydratisierter Form erhalten wird.

**Claims**

1. Method for preparing bismuth tris-trifluoromethanesulphonate, **characterised in that** trifluoromethanesulphonic acid $CF_3SO_3H$ is made to react with bismuth trioxide $Bi_2O_3$ in a medium which comprises a water/alcohol mixture, and **in that** the bismuth tris-fluoromethanesulphonate obtained is recovered.

2. Method for preparing bismuth tris-trifluoromethanesulphonate according to claim 1, **characterised in that** the alcohol used in the reaction medium is an alcohol which is miscible with water and has 1 to 3 carbon atoms.

3. Method for preparing bismuth tris-trifluoromethanesulphonate according to claim 1 or claim 2, **characterised in that** the alcohol used in the reaction medium is ethanol.

4. Method for preparing bismuth tris-trifluoromethanesulphonate according to any of the claims 1 to 3, **characterised in that** the volume ratio alcohol/water within the reaction medium is initially between 50 : 50 and 90 : 10.

5. Method for preparing bismuth tris-trifluoromethanesulphonate according to any of the claims 1 to 4, **characterised in that** the volume ratio alcohol/water within the reaction medium is initially between 60 : 40 and 80 : 20.

6. Method for preparing bismuth tris-trifluoromethanesulphonate according to any of the claims 1 to 5, **characterised in that** the trifluoromethanesulphonic acid and the bismuth trioxide are used with an initial molar ratio $CF_3SO_3H/Bi_2O_3$ between 6 : 1 and 6.5 : 1.

7. Method for preparing bismuth tris-trifluoromethanesulphonate according to any of the claims 1 to 6, **characterised in that** the initial content of $Bi_2O_3$ within the reaction medium is between 40 and 80 g/l.

8. Method for preparing bismuth tris-trifluoromethanesulphonate according to any of the claims 1 to 7, **characterised in that** the initial concentration of triflic acid within the reaction medium is between 70 and 150 g/l.

9. Method for preparing bismuth tris-trifluoromethanesulphonate according to any of the claims 1 to 8, **characterised in that** the reaction of the triflic acid with the bismuth trioxide is conducted at a temperature between 40 and 75°C.

10. Method for preparing bismuth tris-trifluoromethanesulphonate according to any of the claims 1 to 9, **characterised in that** the said bismuth tris-trifluoromethanesulphonate recovered is obtained in a hydrated form.